# EUROPEAN PATENT SPECIFICATION

(11) **EP 1 504 762 B1**
(45) Date of publication and mention of the grant of the patent: **04.08.2010**
(21) Application number: 03723352.5
(22) Date of filing: 13.05.2003
(51) Int. Cl.: A61K 33/24, A61K 31/4402, A61P 25/14, A61P 25/28, A61P 43/00, C07D 213/79

(54) **MEDICINE FOR PREVENTION OF AND TREATMENT FOR FAMILIAL AMYLOID POLYNEUROPATHY**
MEDIKAMENT ZUR PRÄVENTION UND BEHANDLUNG VON FAMILIÄRER AMYLOID POLYNEUROPATHIE
MEDICAMENT POUR PREVENIR ET TRAITER LA POLYNEUROPATHIE AMYLOÏDE FAMILIALE

(30) Priority: 14.05.2002 JP 2002137944
(43) Date of publication of application: 09.02.2005
(73) Proprietor: NIPRO CORPORATION, Osaka-shi, Osaka 531-8510 (JP)
(72) Inventor: KAI, Hirofumi, Kumamoto-shi, Kumamoto 862-0949 (JP); ANDO, Yukio, Kumamoto-shi , Kumamoto 861-5514 (JP)
(74) Representative: von Kreisler Selting Werner
(86) International application number: PCT/JP2003/005963
(87) International publication number: WO 2003/094934

(56) References cited:
- EP-A1- 0 611 570
- WO-A-98/39967
- WO-A1-00/12094
- WO-A1-00/12095
- WO-A1-00/12102
- WO-A1-00/41686
- WO-A1-97/46244
- WO-A1-98/39967
- WO-A1-99/07387
- WO-A2-00/15211
- US-A- 5 677 461
- MIKAMI F ET AL: "Chromium ion suppresses TTR amyloidogenesis" MOLECULAR BIOLOGY OF THE CELL, BETHESDA, MD, US, vol. 13, no. SUPPL, 2002, page 514A, XP008087505 ISSN: 1059-1524
- BENSON M.D.: 'Encyclopedia of Life Sciences', 01 January 2001, JOHN WILEY & SONS; LTD article 'Amyloidosis', pages 1 - 8, XP007907111

## Description

### Technical Field

The present invention relates to a medicine for the prophylaxis and/or treatment of amyloidosis., in particular, familial amyloid polyneuropathy.

### Background Art

Amyloidosis refers to a group of diseases wherein a protein having a β-sheet structure polymerizes to form insoluble fibrils called amyloid (amyloid), which deposit in the body and cause tissue injuries. German pathologist Virchow discovered that tissue specimens of amyloidosis stain purple with iodine. From this result, Virchow considered the substance that had deposited on the tissue as a polysaccharide, named this as "starch-like substance", namely "amyloid", and proposed to call the pathologic condition caused by amyloid deposition as amyloidosis. By subsequent studies, it has been found that the major component of amyloid is the protein that forms the fiber thereof, and that amyloid contains the serum amyloid P component, glycosaminoglycan and the like. At present, amyloid is defined as "comprising an aggregate of non-branched fibrils of 8-15 nm width that stains orange-red with Congo Red Stain and exhibits green vividly shining double refraction when examined under the polarization microscope".

The illness caused by amyloid, that is, amyloidosis, includes various diseases such as Alzheimer's disease, Creutzfeldt-Jacob disease (mad cow disease), Huntington's disease or hereditary diseases. In particular, attention-attracting familial amyloid polyneuropathy (FAP) is a form of amyloidosis characterized by systemic amyloid deposition in organs such as peripheral nerves, autonomic nerves, kidneys, skin and mucosa, and showing introgression by autosomal dominant inheritance. Since a case of FAP was reported for the first time in a Portuguese in 1952, similar case reports have been presented from worldwide. The amyloid fiber that causes FAP is composed of a mutant of transthyretin (TTR), which is a serum protein. The normal TTR usually occurs as a tetramer, whereas mutant TTRs have been shown to be low in structural stability and likely to undergo a structural change from the tetramer to the monomer.

A form of FAP of relatively high incidence in our country is type I FAP, which has a mutant TTR of the Val30Met type as the causal protein. It is said that mutant TTR proteins are lower in structural stability than normal TTR proteins, and that the β-sheet structures in the molecule thereof associate with each other to form insoluble amyloid fiber and deposit in tissue. Major symptoms of type I FAP are multiple neurosis accompanied by sensory disorders that ascend laterally symmetrically from the lower extremity ends, and autonomic disorders (alternating diarrhea and constipation, orthostatic hypotension, dysuria etc.); these are all caused by nerve injuries due to amyloid. Subsequently, amyloid deposition in the heart, kidneys and digestive tract becomes considerable and causes dysfunction of these organs. With no radical therapy to remove once deposited amyloid fiber, FAP is a disease of poor prognosis that develops in the last half of the 20s to the 30s, leads to walking disability in 10 years, and is fatal due to infectious disease, heart failure, renal insufficiency and the like over a course of 10-20 years, and is an intractable disease designated as a specified disease by the Ministry of Health and Welfare.

Currently, although treatment of FAP is conducted mainly by symptomatic therapies, liver transplantation is also adopted as being presumably the most etiotropic therapy. This is a therapeutic method based on the expectation that progression of the condition could be stopped by replacing the patient's liver with a donor liver that produces the normal TTR, because the primary TTR-producing organ is the liver. However, this therapy is not a generally adoptable therapeutic method because an additional burden is exerted on the donor.

The object of the present invention resides in providing a medicine for the prophylaxis and/or treatment of amyloidosis. Particularly, the object of the present invention is to provide a medicine for the prophylaxis and/or treatment of familial amyloid polyneuropathy (FAP), and to provide a medicine to prevent the onset of sensory disorders and autonomic disorders caused by amyloidosis, and a medicine for treatment that enables prevention of progression of the above-described sensory disorders and autonomic disorders, etc.

WO-A-98/39967 discloses the use of chromium in the treatment of Alzheimer's disease, known to belong to the group of amyloid diseases.

### Disclosure of the Invention

The present inventors conducted extensive investigations to accomplish the above-described object, and found that compounds containing the trivalent chromium ion, such as fatty acid chromium salts, have an excellent effect as a medicine for the prophylaxis and/or treatment of familial amyloid polyneuropathy and completed the present invention.

Accordingly, the present invention provides the following:
(1) A compound comprising a trivalent chromium ion for use in the prophylaxis and/or treatment of familial amyloid polyneuropathy
(2) The compound of item 1, wherein the compound comprising the trivalent chromium ion is a fatty acid chromium salt
(3) The compound of item 2, wherein the fatty acid is picolinic acid;
(4) A compound comprising a trivalent chromium ion for use in suppressing decomposition of a mutant transthyretin in familial amyloid polyneuropathy;
(5) A pharmaceutical comprising the compound of any one of items 1 to 3 for use in the prophylaxis and/or treatment of familial amyloid polyneuropathy;
(6) A pharmaceutical comprising a trivalent chromium ion for use in suppressing decomposition of a mutant transthyretin in familial amyloid polyneuropathy;
(7) The pharmaceutical of item 5 or 6 further comprising additives selected from fillers, disintegrants, disintegration aids, binders, lubricants, coating agents, dyes, diluents, substrates, solvents, solubilizers, isotonizing agents, pH regulators, stabilizers, propellants and adhesives, or two or more thereof;
(8) The pharmaceutical of item 5 or 6 in the form of a tablet, capsule, powder, fine granule, granule, liquid, syrup, injection, drip infusion, suppository, inhalant, eye drop, nasal drop, ointment, cream, plaster, transdermally absorbable agent or transmucosally absorbable agent.

In the prophylaxis and/or therapy of familial amyloid polyneuropathy the compound containing the trivalent chromium ion is administered in a prophylactically and/or therapeutically effective amount to a mammalian animal, including human, For the suppression of the decomposition of mutant transthyretin in familial amyloid polyneuropathy a prophylactically and/or therapeutically effective amount of the compound that contains the trivalent chromium ion is administered to a mammalian animal, including a human, are provided by the present invention.

As an active ingredient for the pharmaceutical of the present invention, one or two kinds or more of a compound that contains the trivalent chromium ion can be used. Although the kind of the compound that contains the trivalent chromium ion is not limited, the compound is preferably a fatty acid salt, for example, so that it is readily absorbable from the gastrointestinal tract. As examples of the salt-forming fatty acid, butyric acid, picolinic acid, pyruvic acid etc., which are readily metabolizable via *in vivo* metabolic pathways, are preferred, which, however, are not to be construed as limiting. Of these, picolinic acid, which is non-toxic, and whose rate of gastrointestinal absorption is rapid, is most preferable. Also, as the pharmaceutical of the present invention, an optionally chosen mixture containing a compound that contains the trivalent chromium ion may also be used. Examples of such a mixture include mixtures like natural product extracts and beer fermentation cake.

Although there is no reason for adhering to any particular theory, the action mechanism of the pharmaceutical of the present invention can be explained as follows. Transthyretin (TTR), which is a serum protein, occurs as a tetrameric protein in the serum, and this tetramer is decomposed to monomers by the action of an unidentified catalyst or enzyme and the like. The majority of these monomers are decomposed, some of which go into *in vivo* metabolic pathways and is excreted as urine to outside of the body; some of the monomers undergo rearrangement to form a β-sheet structure, and this β-sheet structure polymerizes linearly to produce insolubilized fibrous amyloid fiber. Mutant transthyretin is prone to be decomposed from the tetrameric protein to the monomer; as a result, amyloid fiber production increases.

The present inventors conducted research into the mechanism by which amyloid fiber is produced from transthyretin, and confirmed the presence of individuals that do not develop FAP despite that they have mutant transthyretin. Hence, on the basis of the idea that environmental factors are involved in the production of the above-described amyloid fiber, the inventors ascertained that metal ions, out of environmental factors, are profoundly involved in amyloid fiber production. For example, as shown in the Examples, the aluminum ion possesses an action to promote the reaction of decomposing the transthyretin tetramer to the monomer. On the other hand, the trivalent chromium ion possesses an action to inhibit the reaction of decomposing the transthyretin tetramer to produce the monomer; as a result, FAP can be prevented and/or treated by suppressing the reaction of producing amyloid fiber from mutant transthyretin.

For example, for mildly symptomatic patients out of patients who have already developed FAP, progression and exacerbation of symptoms can be prevented by administering the pharmaceutical of the present invention. There are some cases wherein a therapeutic effect can be expected even in seriously symptomatic patients. Also, individuals having mutant transthyretin are potential patients who are very likely to develop FAP with aging; onset of FAP can be prevented by administering the pharmaceutical of the present invention.

As the pharmaceutical of the present invention, a compound that contains the trivalent chromium ion, which is an active ingredient, may be used as is; however, usually, it is desirable that a dosage form of a pharmaceutical composition containing both a compound that contains the trivalent chromium ion or a mixture containing it, which is an active ingredient, and one or two kinds or more of additives for pharmaceutical making, be prepared and used.

In preventing and treating FAP, it is also desirable that the pharmaceutical of the present invention be used in combination not only with the trivalent chromium ion, but also with a non-steroidal anti-inflammatory drug that stabilizes the transthyretin tetramer, for example, diflunisal.

As examples of a composition for pharmaceutical use suitable for oral administration, tablets, capsules, powders, fine granules, granules, liquids, and syrups and the like can be mentioned; as examples of a pharmaceutical composition suitable for parenteral administration, injections, drip infusions, suppositories, inhalants, eye drops, nasal drops, ointments, creams, plasters, transdermally absorbable agents, or transmucosally absorbable agents and the like can be mentioned. As examples of additives for preparation making used for production of the above-described pharmaceutical composition, fillers such as lactose and oligosaccharides, disintegrants or disintegration aids, binders, lubricants, coating agents, dyes, diluents, substrates, solvents or solubilizers, isotonizing agents, pH regulators, stabilizers, propellants, and adhesives and the like can be mentioned; these can be appropriately selected according to the form of the pharmaceutical composition by those skilled in the art, and can also be used in combination of two kinds or more.

As a preferred form of the pharmaceutical of the present invention, tablets can be mentioned. Tablet diameter is 2-10 mm or so and thickness is 1~5 mm or so; an active ingredient can be contained at 0.01% by weight or more, preferably 0.1-10% by weigh, per 100% by weight of the tablets. Note that as a medicine that contains chromium picolinate, for example, "Yeast Free Chromium Picolinate" is commercially available as a nutritional supplement in the form of tablets from Vitamin World (NY, USA) Company; this tablet can also be used as is as the pharmaceutical of the present invention.

The dosage and administration frequency and the like for the pharmaceutical of the present invention are not subject to limitation, and can be appropriately selected according to conditions such as patient age, body weight and sex, and the kind and seriousness of the disease, the purpose of prophylaxis or treatment, and the like. Usually, in the case of oral administration, the dosage is 0.01 g to 1 g or so per day for an adult as the amount of active ingredient; the above-described dosage may be administered in several divided portions in a day.

### Brief Description of the Drawings

Figure 1 is a drawing showing that the pharmaceutical of the present invention possesses an action to suppress the decomposition from the transthyretin tetramer to the transthyretin monomer, and that on the other hand, aluminum possesses an action to promote the decomposition.
Figure 2 is a drawing showing that the pharmaceutical of the present invention possesses an action to suppress the decomposition from the normal transthyretin tetramer to the transthyretin monomer, and to suppress amyloid fiber production.
Figure 3 is a drawing showing that the pharmaceutical of the present invention possesses an action to suppress the decomposition from a mutant transthyretin tetramer to the transthyretin monomer, and to suppress amyloid fiber production
Figure 4 is a drawing showing that the pharmaceutical of the present invention possesses an action to increase the thermal stability during structural conversion from the transthyretin tetramer to the transthyretin monomer.
Figure 5 is a drawing showing that the pharmaceutical of the present invention acts to increase the thermal stability during structural conversion from the transthyretin tetramer to the transthyretin monomer, and to stabilize the transthyretin tetramer structure, even under acidic conditions that destabilize the transthyretin tetramer structure.
Figure 6 is a drawing showing that the pharmaceutical of the present invention possesses an action to increase the stability of a mutant transthyretin tetramer structure in vivo.
Figure 7 is a drawing showing that the pharmaceutical of the present invention possesses an action to suppress amyloid fiber production by transthyretin, in cooperation with thyroid hormone, which stabilizes the transthyretin tetramer.

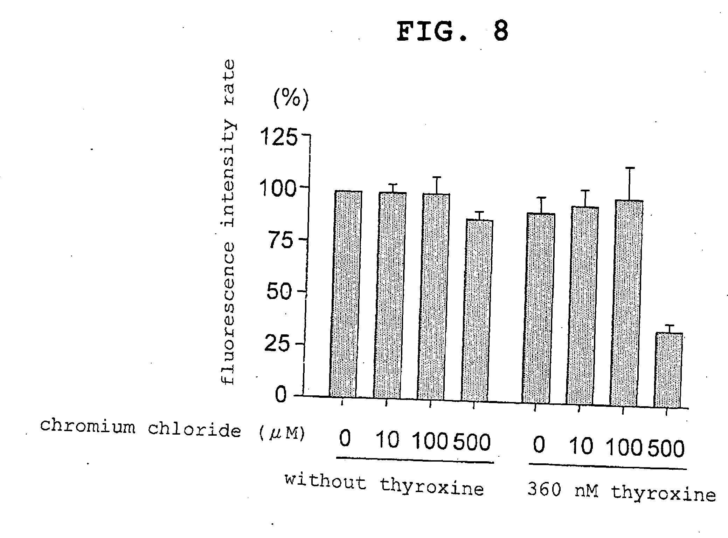

Figure 8 is a drawing showing that the pharmaceutical of the present invention possesses an action to suppress amyloid fiber production by transthyretin, in cooperation with thyroid hormone, which stabilizes a recombinant transthyretin tetramer.

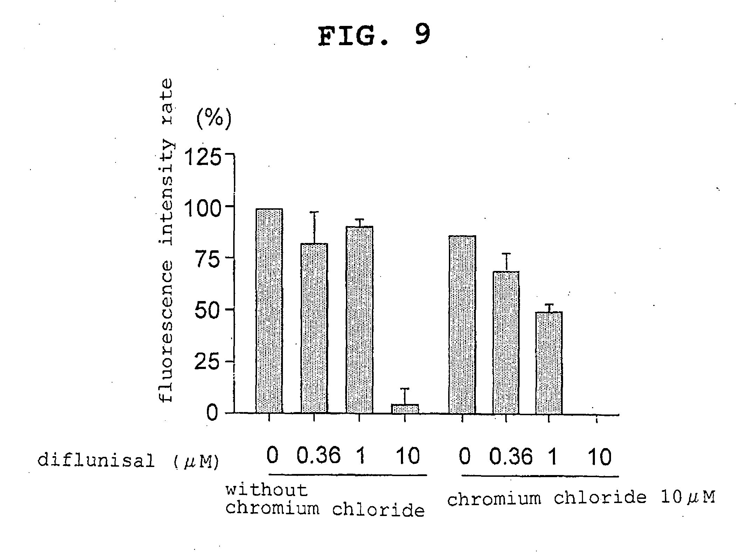

Figure 9 is a drawing showing that the pharmaceutical of the present invention possesses an action to suppress amyloid fiber production by transthyretin, in cooperation with the anti-inflammatory drug diflunisal, which stabilizes the transthyretin tetramer.

### Best Mode for Embodying the Invention

The present invention is hereinafter described more specifically by means of examples, but the scope of the present invention is never limited to the following examples.

### [Example 1]

### (1) Amyloid fiber production test

Using transthyretin purified from human serum as a transthyretin-containing solution, the purity thereof was confirmed by electrophoresis. The transthyretin-containing solution (10 mg/mL) was dissolved in a buffer solution to obtain a concentration of 0.2 mg/mL, and chromium chloride (80 µg/mL) was added. The pH of the buffer solution was adjusted to a pH necessary for each experiment using sodium hydroxide or hydrochloric acid. The above-described solution was incubated at 37°C to produce amyloid fiber.

### (2) Measurement of amount of amyloid fiber produced

Using Thioflavine T (Wako Pure Chemical Industries, Ltd.), which is a fluorescence probe that selectively binds to polymerized β-sheets, the amount of amyloid fiber produced was measured. As a measurement sample, the transthyretin-containing solution after incubation was diluted to obtain a transthyretin concentration of 2.5 µg/mL, supplemented with 10 µM Thioflavine T and 50mM glycine, adjusted to pH 9.0, and used. Within 1 minute after preparation of the measurement sample, fluorescence at 482 nm excited by 450 nm excitation light was measured with the fluorescence spectrophotometer F-4500 at 25°C using 1 ml of the sample. Note that both for the excitation side and for the fluorescence side, the measurement was conducted with a 5 nm slit width.

### (3) Measurement of quantitative ratio of transthyretin tetramer and transthyretin monomer

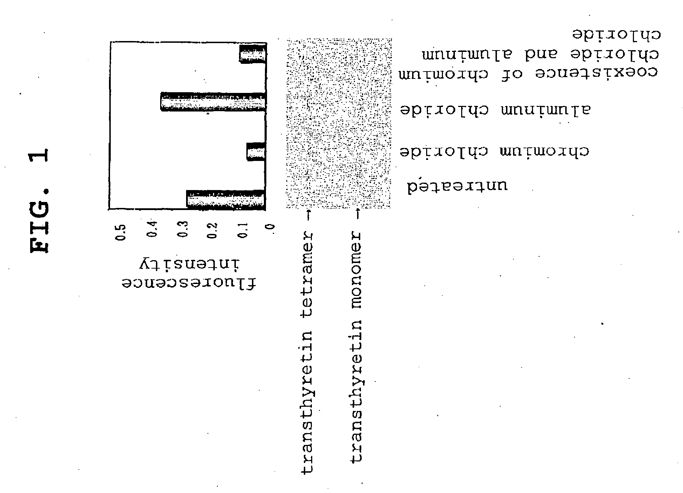

The transthyretin tetramer and the transthyretin monomer were separated by gel separation chromatography, immersed in the Coomassie Brilliant Blue stain, and examined for color developing intensity. Likewise, measurements were conducted for a comparative example wherein the metal compound added to the solution for incubation was changed from chromium chloride to aluminum chloride, and for a control not formulated with chromium chloride. Also, another test was conducted in the same manner, except that the metal compound added to the solution for incubation was replaced with 80 µg/mL chromium chloride and 80 µg/mL aluminum chloride (chromium chloride and aluminum chloride coexisted). Measurement results of fluorescence intensity and the quantitative ratio of the transthyretin tetramer and the transthyretin monomer are shown in Figure 1.

### [Example 2]

### (1) Measurement of changes in thermal stability of transthyretin in the presence of the trivalent chromium ion

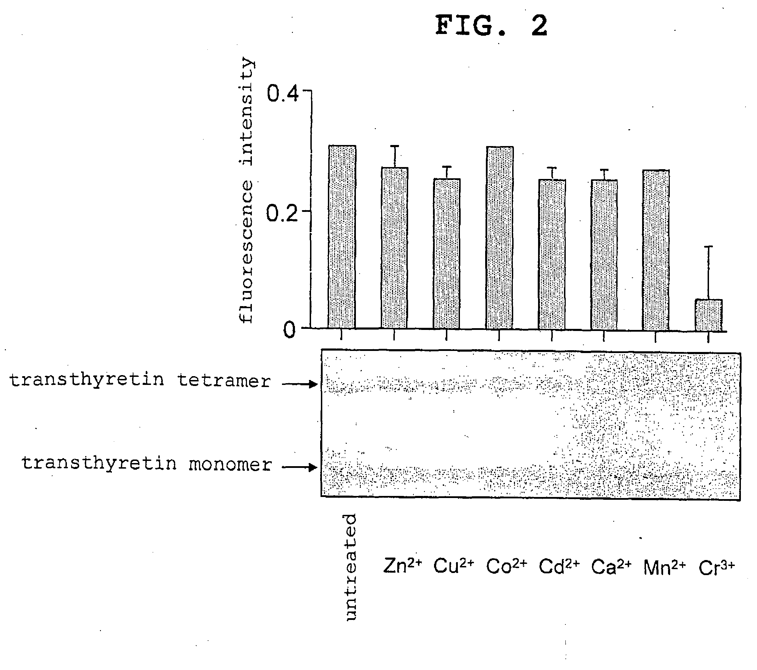

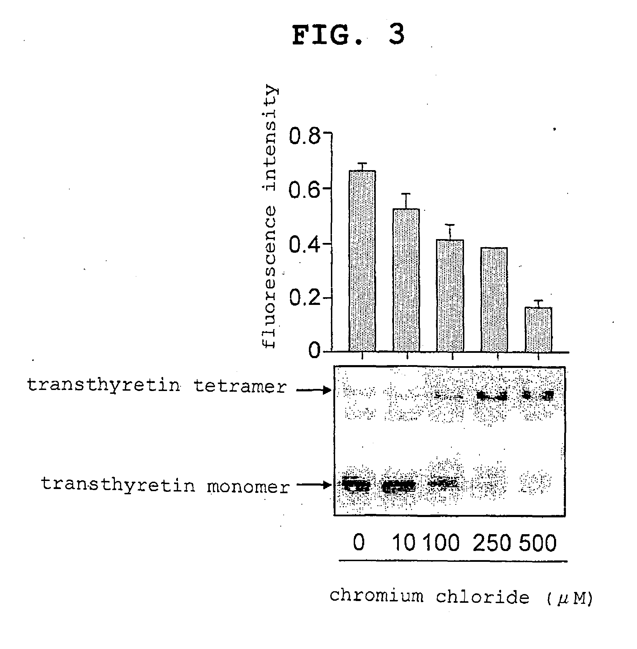

Using a differential scanning calorimeter, changes in the thermal stability during structural conversion from the transthyretin tetramer to the transthyretin monomer in the presence of the trivalent chromium ion were measured. As a measurement sample, a transthyretin-containing solution, prepared to obtain a concentration of 25 µM using 150 mM sodium chloride containing 500 µM chromium chloride and a 20 mM phosphate buffer solution, was used. The pH of the buffer solution was adjusted to a pH necessary for each experiment, using sodium hydroxide or hydrochloric acid. Measurements were conducted by injecting 1.5 ml of the sample solution to a measurement cell after degassing at room temperature for 5 minutes. The measurement conditions involved the use of a model MC-2 differential scanning calorimeter manufactured by MicroCal at a scanning rate of 1°C/min in a temperature range of 15°C to 115°C; during the measurement, the system was pressurized with 2 atm of nitrogen to prevent bubbling due to heat. Note that the measurement cell was washed with concentrated nitric acid for each sample. Measurement results of the amount of heat and transition temperature are shown in Figure 2 and Figure 3.

### [Example 3]

### (1) Test of administration of trivalent chromium ion to transthyretin transgenic mouse

Administration of the trivalent chromium ion to a transgenic mouse incorporating a human mutant transthyretin gene having the 30th valine residue of transthyretin mutated to a methionine residue was conducted by intraperitoneally injecting 3.5 µg of a chromium chloride solution per gram of mouse weight, which is the one-sixth dosage of the LD50 value, daily. One week after administration initiation, mouse blood was recovered from the tail vein and allowed to stand at 4°C overnight, and the serum component was isolated and used as the sample.

### (2) Measurement of stability of transthyretin tetramer of transthyretin transgenic mouse

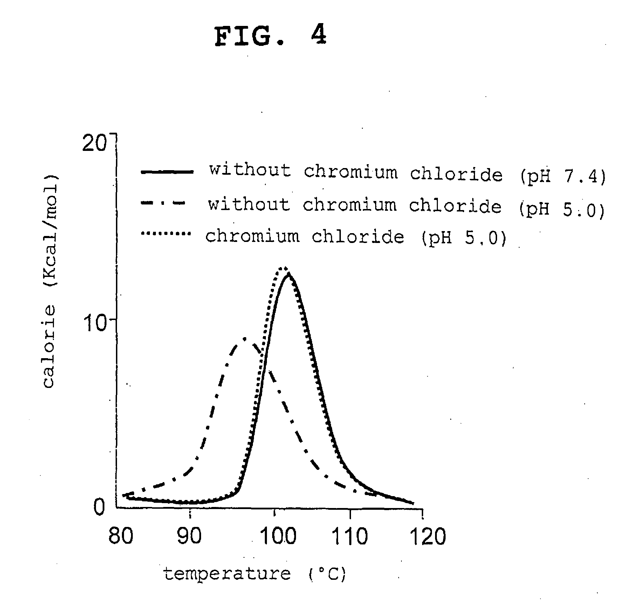

The stability of the transthyretin tetramer in a 60 µg sample recovered from the blood of the transgenic mouse was measured using Western blotting. The primary antibody used for Western blotting was an anti-human mutant transthyretin antibody (1:2000); after a primary antibody reaction, a secondary antibody reaction was conducted using a peroxidase-labeled anti-rabbit secondary antibody (1:10000). For detection of the antibody reaction, the ECL reagent was used. Results of detection of the transthyretin tetramer of the transgenic mouse are shown in Figure 4.

### [Example 4]

### (1) Amyloid fiber production test

A transthyretin-containing solution was dissolved in a buffer solution to obtain a concentration of 0.2 mg/mL, 0, 10 or 50 µM chromium chloride was added, and 0 or 360 nM thyroid hormone was added to each solution. The pH of the buffer solution was adjusted to a pH necessary for each experiment using sodium hydroxide or hydrochloric acid. The above-described solution was incubated at 37°C to produce amyloid fiber.

### (2) Measurement of amount of amyloid fiber produced

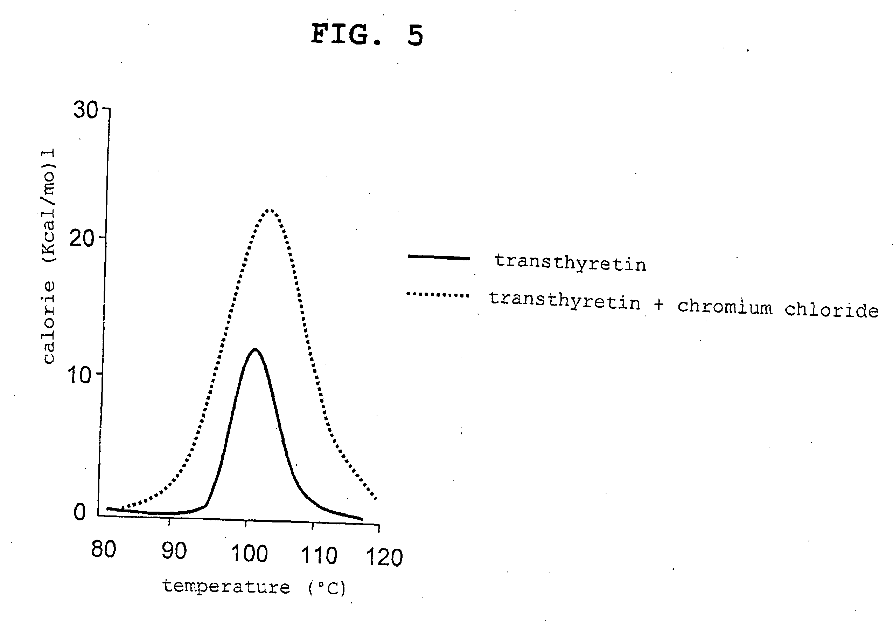

Using Thioflavine T (Wako Pure Chemical Industries, Ltd.), the amount of amyloid fiber produced was measured. As a measurement sample, the transthyretin-containing solution after incubation was diluted to obtain a transthyretin concentration of 2.5 µg/mL, supplemented with 10 µM Thioflavine T and 50mM glycine, adjusted to pH 9.0, and used. Within 1 minute after preparation of the measurement sample, fluorescence at 482 nm excited by 450 nm excitation light was measured with the fluorescence spectrophotometer F-4500 at 25°C using 1 ml of the sample. Note that both for the excitation side and for the fluorescence side, the measurement was conducted with a 5 nm slit width. Measurement results of fluorescence intensity are shown in Figure 5.

### [Example 5]

### (1) Amyloid fiber production test

A recombinant transthyretin-containing solution (10 mg/ml) was dissolved in a buffer solution to obtain a concentration of 0.2 mg/mL, 0, 10, 100 or 500 µM chromium chloride was added, and 0 or 360 nM thyroid hormone was added to each solution. The pH of the buffer solution was adjusted to a pH necessary for each experiment using sodium hydroxide or hydrochloric acid. The above-described solution was incubated at 37°C to produce amyloid fiber.

### (2) Measurement of amount of amyloid fiber produced

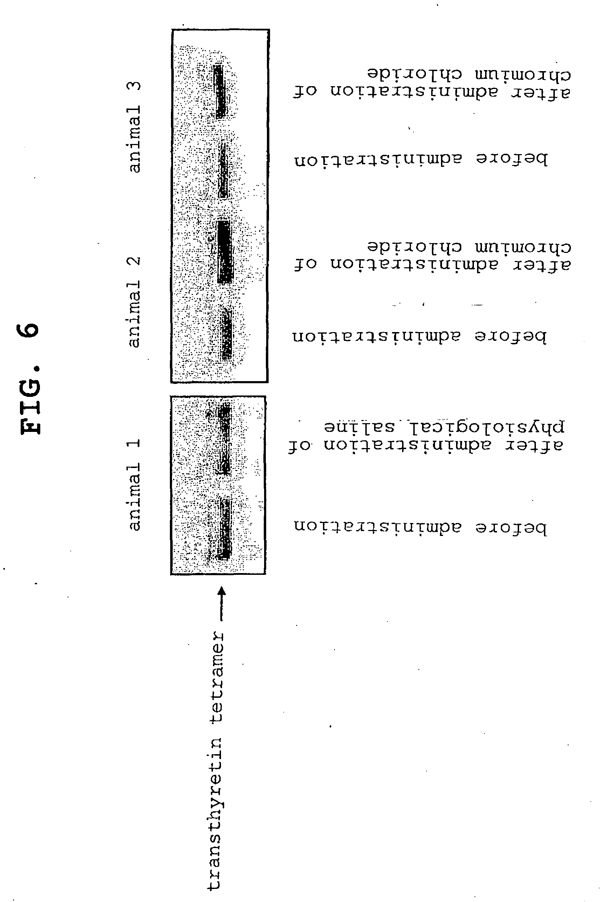

Using Thioflavine T (Wako Pure Chemical Industries, Ltd.), the amount of amyloid fiber produced was measured. As a measurement sample, the transthyretin-containing solution after incubation was diluted to obtain a transthyretin concentration of 2.5 µg/mL, supplemented with 10 µM Thioflavine T and 50 mM glycine, adjusted to pH 9.0, and used. Within 1 minute after preparation of the measurement sample, fluorescence at 482 nm excited by 450 nm excitation light was measured with the fluorescence spectrophotometer F-4500 at 25°C using 1 ml of the sample. Note that both for the excitation side and for the fluorescence side, the measurement was conducted with a 5 nm slit width. Measurement results of fluorescence intensity are shown in Figure 6.

### [Example 6]

### (1) Amyloid fiber production test

A transthyretin-containing solution was dissolved in a buffer solution to obtain a concentration of 0.2 mg/mL, 0, 0.36, 1 or 10 µM diflunisal was added, and 0 or 10 µM chromium chloride was added to each solution. The pH of the buffer solution was adjusted to a pH necessary for each experiment using sodium hydroxide or hydrochloric acid. The above-described solution was incubated at 37°C to produce amyloid fiber.

### (2) Measurement of amount of amyloid fiber produced

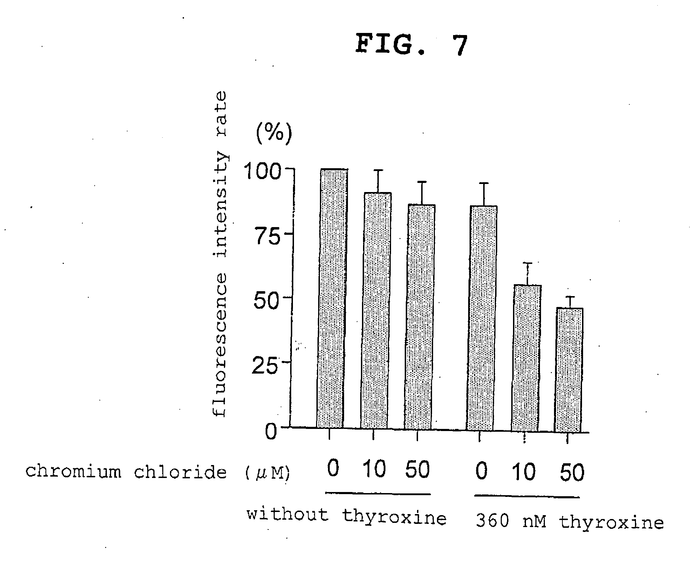

Using Thioflavine T (Wako Pure Chemical Industries, Ltd.), the amount of amyloid fiber produced was measured. As a measurement sample, the transthyretin-containing solution after incubation was diluted to obtain a transthyretin concentration of 2.5 µg/mL, supplemented with 10 µM Thioflavine T and 50mM glycine, adjusted to pH 9.0, and used. Within 1 minute after preparation of the measurement sample, fluorescence at 482 nm excited by 450 nm excitation light was measured with the fluorescence spectrophotometer F-4500 at 25°C using 1 ml of the sample. Note that both for the excitation side and for the fluorescence side, the measurement was conducted with a 5 nm slit width. Measurement results of fluorescence intensity are shown in Figure 7.

### Industrial Applicability

The pharmaceutical of the present invention is useful and highly effective as a medicine for the prophylaxis and/or treatment of familial amyloid polyneuropathy (FAP), for which no therapies are available other than symptomatic therapies and liver transplantation.

## Claims

1. A compound comprising a trivalent chromium ion for use in the prophylaxis and/or treatment of familial amyloid polyneuropathy.

2. The compound of claim 1, wherein the compound comprising the trivalent chromium ion is a fatty acid chromium salt.

3. The compound of claim 2, wherein the fatty acid is picolinic acid.

4. A compound comprising a trivalent, chromium ion for use in suppressing decomposition of a mutant transthyretin in familial amyloid polyneuropathy.

5. A pharmaceutical comprising the compound of any one of claims 1 to 3 for use in the prophylaxis and/or treatment of familial amyloid polyneuropathy.

6. A pharmaceutical comprising a trivalent chromium ion for use in suppressing decomposition of a mutant transthyretin in familial amyloid polyneuropathy.

7. The pharmaceutical of claim 5 or 6 further comprising additives selected from fillers, disintegrants, disintegration aids, binders, lubricants, coating agents, dyes, diluents, substrates, solvents, solubilizers, isotonizing agents, pH regulators, stabilizers, propellants and adhesives, or two or more thereof.

8. The pharmaceutical of claim 5 or 6 in the form of a tablet, capsule, powder, fine granule, granule, liquid, syrup, injection, drip infusion, suppository, inhalant, eye drop, nasal drop, ointment, cream, plaster, transdermally absorbable agent or transmucosally absorbable agent.

9. Use of a compound comprising a trivalent chromium ion for the manufacture of a medicament for the prophylaxis and/or treatment of familial amyloid polyneuropathy.

## Patentansprüche

1. Verbindung, die ein dreiwertiges Chrom-Ion umfasst, zur Verwendung bei der Prophylaxe und/oder Behandlung von familiärer Amyloid-Polyneuropathie.

2. Verbindung gemäß Anspruch 1, wobei die Verbindung, die das dreiwertige Chrom-Ion umfasst, ein Fettsäure-Chromsalz ist.

3. Verbindung gemäß Anspruch 2, wobei es sich bei der Fettsäure um Picolinsäure handelt.

4. Verbindung, die ein dreiwertiges Chrom-Ion umfasst, zur Verwendung bei der Unterdrückung der Zersetzung eines mutanten Transthyretins bei familiärer Amyloid-Polyneuropathie.

5. Medikament, das die Verbindung gemäß einem der Ansprüche 1 bis 3 umfasst, zur Verwendung bei der Prophylaxe und/oder Behandlung von familiärer Amyloid-Polyneuropathie.

6. Medikament, das ein dreiwertiges Chrom-Ion umfasst, zur Verwendung bei der Unterdrückung der Zersetzung eines mutanten Transthyretins bei familiärer Amyloid-Polyneuropathie.

7. Medikament gemäß Anspruch 5 oder 6, das weiterhin Additive umfasst, die aus Füllstoffen, Sprengmitteln, Sprenghilfsmitteln, Bindemitteln, Gleitmitteln, Überzugsmitteln, Farbstoffen, Verdünnungsmitteln, Grundlagen, Lösungsmitteln, Lösungsvermittlern, Isotonisierungsmitteln, pH-Regulatoren, Stabilisatoren, Treibmitteln und Klebstoffen oder zweien oder mehreren davon ausgewählt sind.

8. Medikament gemäß Anspruch 5 oder 6 in Form einer Tablette, Kapsel, eines Pulvers, Feingranulats, Granulats, einer Flüssigkeit, eines Sirups, einer Injektionslösung, Infusionslösung, eines Suppositoriums, Inhalationsmittels, Augentropfen, Nasentropfen, einer Salbe, Creme, eines Pflasters, transdermal resorbierbaren Mittels oder transmukosal resorbierbaren Mittels.

9. Verwendung einer Verbindung, die ein dreiwertiges Chrom-Ion umfasst, zur Herstellung eines Medikaments zur Prophylaxe und/oder Behandlung von familiärer Amyloid-Polyneuropathie.

## Revendications

1. Composé comprenant un ion de chrome trivalent pour utilisation dans la prophylaxie et/ou le traitement de la polyneuropathie amyloïde familiale.

2. Composé selon la revendication 1, dans lequel le composé comprenant l'ion de chrome trivalent est un sel de chrome d'acide gras.

3. Composé selon la revendication 2, dans lequel l'acide gras est l'acide picolinique.

4. Composé comprenant un ion de chrome trivalent pour utilisation dans la suppression de la décomposition d'une transthyrétine mutante dans la polyneuropathie amyloïde familiale.

5. Produit pharmaceutique comprenant le composé selon l'une quelconque des revendications 1 à 3, pour utilisation dans la prophylaxie et/ou le traitement de la polyneuropathie amyloïde familiale.

6. Produit pharmaceutique comprenant un ion de chrome trivalent pour utilisation dans la suppression de la décomposition d'une transthyrétine mutante dans la polyneuropathie amyloïde familiale.

7. Produit pharmaceutique selon la revendication 5 ou 6, comprenant en outre des additifs choisis parmi les charges, les délitants, les adjuvants d'aide au délitement, les liants, les lubrifiants, les agents d'enrobage, les colorants, les diluants, les substrats, les solvants, les agents de solubilisation, les agents d'isotonisation, les régulateurs de pH, les agents de stabilisation, les gaz propulseurs et les adhésifs, ou deux ou plus de deux de ceux-ci.

8. Produit pharmaceutique selon la revendication 5 ou 6, sous la forme d'un comprimé, d'une capsule, d'une poudre, de granulés fins, de granulés, d'un liquide, d'un sirop, d'une injection, d'une perfusion goutte-à-goutte, d'un suppositoire, d'un inhalant, de gouttes ophtalmologiques, de gouttes nasales, d'une pommade, d'une crème, d'un plâtre, d'un agent pouvant être absorbé à travers la peau ou d'un agent pouvant être absorbé à travers les muqueuses.

9. Utilisation d'un composé comprenant un ion de chrome trivalent pour la fabrication d'un médicament destiné à la prophylaxie et/ou au traitement de la polyneuropathie amyloïde familiale.
